# EUROPEAN PATENT APPLICATION

(11) **EP 4 000 602 A1**
(43) Date of publication of application: **25.05.2022**
(21) Application number: 20207808.5
(22) Date of filing: 16.11.2020
(51) Int. Cl.: A61K 9/107, A61K 31/196, A61K 31/616, A61K 33/30, A61K 47/10, A61K 47/44, A61P 29/00

(54) **NOVEL ZN(II)-NSAID-COMPLEX COMPOSITION**

(71) Applicant: concrete flowers GmbH, 2603 Felixdorf (AT)
(72) Inventor: Seiler, Friederike, 1040 Wien (AT)

(57) **Abstract**

The invention refers to a novel pharmaceutical composition comprising a Zn(II)-complex of a non-steroidal anti-inflammatory drug (NSAID) selected from aspirin (acetylsalicylic acid), flurbiprofen, diclofenac, mefenamic acid, dexibuprofen, and ibuprofen (Zn(II)-NSAID-complex), optionally further comprising nitric oxide (Zn(II)-NO-NSAID-complex), a hydrophilic surfactant, and one or more mucoadhesive agents with increased solubility and permeability, better absorption and a higher AUC. The composition has anti-viral, anti-bacterial and anti-inflammatory properties for the treatment of diseases and disorders related to pain, infection and inflammation.

## Description

### FIELD OF THE INVENTION

The invention refers to a novel pharmaceutical composition with increased solubility and permeability, better absorption and a higher AUC, comprising a Zn(II)-NSAID-complex or a Zn(II)-NO-NSAID-complex of a non-steroidal anti-inflammatory drug (NSAID) selected from aspirin (acetylsalicylic acid), flurbiprofen, diclofenac, mefenamic acid, dexibuprofen, and ibuprofen, a hydrophilic surfactant, and one or more mucoadhesive agents. The composition has anti-viral, anti-bacterial and anti-inflammatory properties and is useful for the treatment of diseases and disorders related to pain, infection and inflammation.

### BACKGROUND OF THE INVENTION

Pain represents a major public health problem worldwide, with chronic pain affecting approximately 27% of the adult population in Europe and more than 100 million adults in the United States. Undertreated acute pain can be associated with an increased risk of deleterious health consequences (e.g. delayed wound healing, immune dysfunction, cardiovascular problems related to the stress response, and respiratory problems, such as pneumonia) and the development of chronic pain. In addition, unrelieved chronic severe pain can negatively impact an individual's quality of life, day-to-day functioning, sleep quality, interpersonal relationships, and work productivity, and is associated with a substantial economic burden.

There are two major options for the pharmacological management of pain: non-steroidal anti-inflammatory drugs (NSAIDs), which act via inhibition of cyclooxygenase (COX) isozymes, and opiate/opioid analgesics.

Non-steroidal anti-inflammatory drugs (NSAIDs) are among the most widely used therapeutics. Through their anti-inflammatory, anti-pyretic and analgesic activities, they represent a choice treatment in various inflammatory diseases such as arthritis, rheumatisms as well as relieving the pains of everyday life. From a historical viewpoint, the first NSAID with therapeutic benefits was aspirin, which has now been used for more than 100 years.

Despite the wide use of NSAIDs over the last century, their mechanism of action was not fully understood until 1971 when Vane identified their molecular target, the COX enzyme (Vane J.R. and Botting R.M., 2003, Thromb Res., 110(5-6):225-8). In the early 1990s, a second isoform (COX-2) was discovered, distinct from the first one, then renamed COX-1. COX-1 and COX-2 are isoenzymes. COX-1 is expressed constitutively in many tissues and prostaglandins produced by COX-1, mediating "housekeeping" functions such as cytoprotection of gastric mucosa, regulation of renal blood flow and platelet aggregation. In contrast, COX-2 is not detected in most normal tissues, but its expression is rapidly induced by stimuli such as proinflammatory cytokines (IL-1b, TNFα), lipopolysaccharides, mitogens and oncogenes (phorbol esters), growth factors (fibroblast growth factor (FGF); platelet-derived growth factor (PDGF), epidermal growth factor (EGF), hormones (luteinizing hormone (LH)) and disorders of water-electrolyte hemostasis, resulting in increased synthesis of PGs in inflamed and neoplastic tissues. Thus, the inducible isozyme has been implicated in pathological processes such as inflammation and various cancer types.

In addition to dose, therapeutic activity and side effects of NSAIDs depend on their absorption, distribution, and elimination. As with any analgesic class, the rate of absorption is a key factor in the selection of an NSAID; those with rapid absorption are preferable for most patients, particularly those with severe or acute pain. The distribution of NSAIDs in injured tissues, blood, and other areas of the body is a particularly crucial consideration for ensuring activity at the site of inflammation, as well as reducing the risk of side effects unrelated to therapeutic activity on COX enzymes throughout the body. NSAIDs can be classified as acidic or non-acidic based on their chemical structure, and the acidity of the drug can have an effect on its distribution. NSAIDs with acidic functional groups (e.g. diclofenac, ibuprofen) and with a high degree of protein binding have been shown to selectively accumulate and persist at sites of inflammation, while non-acidic NSAIDs (e.g. acetaminophen, celecoxib, rofecoxib) tend to be distributed homogenously throughout the body. Acidic NSAIDs with a high degree of protein binding may remain in inflamed tissues or synovial fluid for a longer time than in the plasma. The persistence of certain NSAIDs (e.g. diclofenac, ibuprofen) in the synovial fluid is associated with a sustained therapeutic effect despite relatively rapid clearance from the plasma, vascular wall, and kidneys, indicating that accumulation at sites of inflammation may allow for continued anti-inflammatory and analgesic activity. The localization of COX activity to inflamed tissue and the resulting ability to use lower doses than might otherwise be required also minimizes COX inhibition at sites associated with potential side effects (e.g. hepatic, renal, cardiovascular, and gastrointestinal adverse effects).

Zinc has specific chemical properties which makes it more important than other metals. It is considered one of the metals essential to life; it is difficult to imagine living organisms able to carry out their metabolic activity with its absence. It is the only metal that appears in all enzyme classes with important anti-bacterial and anti-viral effects.

With a small size of zinc ion and a concentrated positive charge (single oxidation state), zinc has a strong binding capacity with an ability to react as a strong Lewis acid. It is soluble in the body at millimolar concentrations, however zinc salts are highly hydrophobic thus leading to reduced solubility of zinc salt complexes.

The positively charged zinc ions interact with proteins, lipids and carbohydrates present in saliva and oral and nasal tissues. Although these interactions are important as shown by Bakar, N.K.A. et al. (1999, The chemical speciation of zinc in human saliva: possible correlation with reduction of the symptoms of the common cold produced by zinc gluconate containing lozenges, Chemical Speciation & Bioavailability, Chem. Speciat. Bioavailab. 11:3, 95-101), they mainly result in a requirement for a much larger concentration (-700-fold) of positively charged zinc in formulations than would be required for biological effects in vitro.

In addition to the concentration of zinc ions, the charge of zinc is important. Analyses of Zn lozenge compositions using solution chemistry methods allows the valid determination of zinc ionic species at physiologic pH 7.4. Neutrally charged zinc has no effect on colds. Negatively charged zinc worsens colds.

A larger correlation between free Zinc ions and efficacy can be obtained by also considering lozenge oral dissolution times, and saliva production during oral dissolution of lozenges, in accordance with Fick's laws of membrane permeability.

Hirt, M. et al. (2000, Zinc nasal gel for the treatment of common cold symptoms: a double-blind, placebo-controlled trial, ENT 79:778-80, 82) and Mossad, S.B. (2003, Effect of zincum gluconicum nasal gel on the duration and symptom severity of the common cold in otherwise healthy adults, QJM 96:35-43) have shown in their studies that ionized zinc has direct antirhinoviral activity, protects cells from damage by viral toxins, and might inhibit the rhinovirus' ability to attach to, enter, and infect human nasal cells. Various compounds of zinc are already used intra-nasally in the treatment of rhinitis. e.g. Wick Erste Abwehr, Zicam^{®} nasal gel using zinc acetate and chelated zinc EDTA.

Apart from the use of Zn lozenge for the treatment of rhinovirus common colds, studies have shown that Zn lozenges have been useful in treating oral, lip and nasal herpes infections with benefit, including reducing occurrence and duration of outbreaks (Eby, G. A. and Halcomb, W. W., 1985, Use of topical zinc to prevent recurrent herpes simplex infection: review of literature and suggested protocols, Med. Hypotheses 17:157-165). ZN(II)-NSAID complexes have already been described, such as for Naproxen (EP0987023A1). However, although these Zn complexes had been developed to provide improved galenic dosage forms, these complexes are still poorly soluble.

Therefore, there is an unmet need to enhance the aqueous solubility and dissolution rate of Zn(II)-NSAID-complexes in order to achieve faster onset of action and to minimize the absorption variability.

### SUMMARY OF THE INVENTION

It is an object of the present invention to provide an NSAID composition with improved solubility, improved dissolution rate, increased and rapid bioavailability.

The problem is solved by the present invention. Preparations comprising the inventive NSAID compositions show prolonged mucoadhesion properties, thereby increasing permeability, specifically resulting in higher AUC and thus less long-term negative side effects, faster onset of action and minimizing absorption variability.
It has surprisingly been found that a pharmaceutical composition comprising
i) a Zn(II)-complex of a non-steroidal anti-inflammatory drug (NSAID) selected from the group consisting of any one of aspirin (acetylsalicylic acid), flurbiprofen, diclofenac, mefenamic acid, dexibuprofen, and ibuprofen (Zn(II)-NSAID-complex), optionally further comprising nitric oxide (Zn(II)-NO-NSAID-complex),
ii) a hydrophilic surfactant, and
iii) one or more mucoadhesive agents;
has high solubility and shows fast bioavailability which is highly important specifically in view of pain treatment.

Poor solubility of Zn(II)-NSAID-complexes and Zn(II)-NO-NSAID-complexes is a main obstacle for onset of action. Dissolution in the environmental lumen is the rate-controlling step in the absorption process. Moreover, low aqueous solubility leads to high variability in absorption specifically after oral administration. The poor aqueous solubility of Zn(II)-NSAID-complexes and Zn(II)-NO-NSAID-complexes typically exhibit dissolution rate limited absorption, thus resulting in permeation rate limited absorption.

The present pharmaceutical composition is improving the bioavailability, specifically oral bioavailability, of the active agents including enhancing solubility and dissolution rate of the poorly water-soluble Zn(II)-NSAID-complex and Zn(II)-NO-NSAID-complex as well as prolonging the period of mucoadhesion to increase permeability and thus resulting in a higher AUC.

Specifically, the hydrophilic surfactant is selected from the group consisting of polyethylene glycols such as PEG 300, PEG 400, PEG15; polyoxyethylene (20) cetyl ether, ethanol, propylene glycol, glycerol, and diethylene glycol monoethyl ether.

Specifically, the Zn(II) compound of the complex is zinc chloride or a Zn(II)-dichloro complex with 1,10-phenanthroline-furoxan. Specifically, the weight ratio of zinc to NSAID is 1:2.

According to an embodiment, the mucoadhesive agent can be any one of the following:
I. natural polymers, specifically hyaluronate, pectin, alginate, xanthin gum, and guar gum,
II. cyclodextrins, specifically α cyclodextrin, β cyclodextrin, γ cyclodextrin, and methylated β-cyclodextrins,
III. hydrogen bond polymers, specifically acrylate, more specifically hydroxylated methacrylate poly(methacrylic acid), butylmethacrylate, poly[acrylic acid-co-(2-ethylhexyl)acrylate-co-methylacrylate], high molecular weight crosslinked polyacrylic acid polymers (Carbopol^{™} (CP)), and polycarbophil (PC),
IV. cationic compounds, specifically poly-l-arginine and l-lysine,
V. non-ionic polymers, specifically hydroxyethyl starch, hydroxypropyl cellulose (HPC), poly(ethylene oxide), polyvinyl alcohol (PVA), polyvinylpyrrolidone (PVP), scleroglucan, polyoxyethylene poly (methacrylic acid co-methyl methacrylate), and
VI. positively charged polymers, specifically chitosan, trimethyl chitosan, aminodextran, dimethylaminoethyl (DMAE), diethylaminoethyl (DEAE), dextran.

In a specific embodiment, the pharmaceutical composition described herein additionally comprises uncomplexed NSAIDs and related compounds, specifically selected from ibuprofen, ibuprofen lysine, ibuprofen arginate, ibuprofen sodium salt, ibuprofen calcium salt, mefenamic acid, sodium mephenamate, mefenamin lysinate, mefenamin arginate, dexibuprofen, dexibuprofen lysine, diclofenac sodium salt, flurbiprofen, flurbiprofen sodium salt, aspirin, aspirin calcium, aspirin sodium salt, and aspirin DL-lysine.

In a further embodiment, the pharmaceutical composition described herein additionally comprises one or more of calcium ascorbate, caffeine, caffeine citrate, chlorpheniramine maleate, D-pseudoephedrine, or pseudoephedrine sulfate.

Herein provided is also an emulsion comprising
a. a Zn(II)-complex of a non-steroidal anti-inflammatory drug (NSAID) selected from the group consisting of any one of aspirin (acetylsalicylic acid), flurbiprofen, diclofenac, mefenamic acid, dexibuprofen, and ibuprofen (Zn(II)-NSAID-complex), optionally further comprising nitric oxide (Zn(II)-NO-NSAID-complex), and a hydrophilic surfactant, and one or more mucoadhesive agents,
b. a co-surfactant,
c. an oil, and
d. an aqueous solution.

Specifically, the emulsion is a water in oil, an oil in water, or a bicontinuous emulsion.

More specifically, the emulsion comprises droplets, with a droplet size in the range of 10 to 1000 nm, specifically of 10 to 500 nm, specifically of 10 to 200 nm, specifically of 10 to 150 nm.

According an embodiment of the invention, the emulsion described herein comprises a co-surfactant which is any one of nonionic tensides, specifically polyoxylglycerides (e.g. Labrasol^{®}, Labrafil^{®}, Gelucire^{®}), ethoxylated glycerides derived from castor oil (e.g. Cremophor EL, RH40, or RH60), esters of fatty acids and alcohols (e.g., Plurol^{®} Oleique CC497, Capryol^{®} 90, Lauroglycol^{™} 90, Mirj^{®} 45, Mirj^{®} 52, Solutol^{®} HS15, Span^{®} 80, Span^{®} 20, Tween^{®} 80, Tween^{®} 20) or sucrose ester-based natural surfactants (e.g. sucrose laurate L-1695, sucrose stearate S-970, sucrose monolaurate + sucrose dilaurate, sucrose laurate D-1214, sucrose myristate C-1416, sucrose palmitate P-1670 -/+ sucrose stearate S-970) or any combination thereof.

According a further embodiment of the invention, the emulsion described herein comprises an oil which is selected from the group of vegetable oils, hydrogenated vegetable oils, or partial glycerides, more specifically corn oil, olive oil, peanut oil, rapeseed oil, sesame oil, soybean oil, coconut, palm seed oil, cottonseed oil, such as AkofineTM, Sterotex^{®}, Dynasan^{®} P60, Softisan^{®} 154, Suppocire^{®}, Cutina^{®} HR, Sterotex^{®} HM, Hydrocote, Lipo, Capmul^{®} MCM, Geleol^{®}, Imwitor^{®} 191, Cutina^{®} GMS, Tegin^{®}, Precirol^{®} ATO 5, Peceol^{®}, Maisine^{®} 35-1, Compritol^{®} 888 ATO.

In a further embodiment, the emulsion contains dexpanthenol.

The pharmaceutical composition or emulsion described herein can be formulated for local administration, preferably for intraoral, oral, dermal, ocular administration, for systemic administration, for rectal administration, for vaginal administration, application to the upper and lower respiratory tract, nasal administration, or pulmonary administration.

In a further embodiment, the pharmaceutical composition, or the emulsion described herein are prepared for administration as a spray, a powder, a gel, an ointment, a cream, a foam, or a liquid solution, a lotion, a gargle solution, an aerosolized powder, an aerosolized liquid formulation, a granule, a capsule, a drop, a tablet, a troche, a syrup, a lozenge, a solid or semi-solid candy, an eye drop, a gum, a suppository, a transdermal patch or a preparation for infusion or injection.

A method for treating pain, inflammation, problems associated to wound healing, and/or infection with gram negative, gram-positive bacteria or yeast, such as but not limited to *Pseudomonas aeruginosa, Staphylococcus aureus, Aspergillus spp., Candida albicans, C. parapsilosis,* is also provided herein, comprising the step of administering to a subject in need thereof a pharmaceutical composition or an emulsion described herein.

Additionally, also the use of the pharmaceutical composition or the emulsion described herein as analgesic, anti-pyretic or anti-inflammatory agent is provided by the present invention.

In a further embodiment, the use of the pharmaceutical composition, or the emulsion described herein is also provided to inhibit growth of gram-positive, gram-negative bacteria or yeasts in a subject.

In a further embodiment, the pharmaceutical composition, or the emulsion described herein is for use in treating pain, inflammation, problems associated to wound healing, and/or infection with gram negative, gram-positive bacteria or yeast, such as but not limited to *Pseudomonas aeruginosa, Staphylococcus aureus, Aspergillus spp., Candida albicans, C. parapsilosis.*

In a further embodiment, the pharmaceutical composition, or the emulsion described herein is for use as analgesic, anti-pyretic or anti-inflammatory agent.

In a further embodiment, the pharmaceutical composition, or the emulsion described herein is for use in inhibiting growth of gram-positive, gram-negative bacteria or yeasts in a subject.

### DETAILED DESCRIPTION

Unless indicated or defined otherwise, all terms used herein have their usual meaning in the art, which will be clear to the skilled person. Specific terms as used throughout the specification have the following meaning.

Unless indicated otherwise, the term "about" as used herein refers to the same value or a value differing by up to +/-5 % of the given value.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

As used herein, the term "subject" or "individual" or "patient" shall refer to a warm-blooded mammalian, particularly a human being or a non-human animal, including e.g., dogs, cats, rabbits, horses, cattle, and pigs.

The term "patient" includes mammalian, specifically human, subjects that receive either prophylactic or therapeutic treatment or are at risk of or diagnosed of a specific disease or disorder, particularly those conditions as further described herein.

The Zinc(II)-NSAID complex and Zn(II)-NO-NSAID-complex comprising acetylsalicylic acid, flurbiprofen, diclofenac, mefenamic acid, dexibuprofen, and ibuprofen has successfully been synthesized herein and combined with a hydrophilic surfactant and one or more mucoadhesive agents to obtain a pharmaceutical composition.

This pharmaceutical composition was found to be highly improved, thus having a more potent anti-inflammatory activity, improved solubility, improved dissolution rate, increased and rapid bioavailability as well as prolonged mucoadhesion, thereby increasing permeability, specifically resulting in higher AUC and thus less long-term negative side effects, faster onset of action and minimizing absorption variability compared to the respective NSAIDs not being formulated as described herein. In addition, the composition has higher analgesic properties.

**Ibuprofen** (IB) ([2RS]-1[4-[2-methyl propyl] phenyl] propionic acid) is characterized by the structure 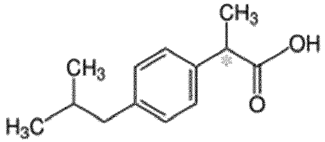 and is a nonsteroidal anti-inflammatory drug (NSAID) that has been widely used in the treatment of mild to moderate pain and fever. It is a nonselective inhibitor of cyclooxygenase-1 (COX-1) and COX-2. Although its anti-inflammatory properties may be weaker than those of some other NSAIDs, it has a prominent analgesic and antipyretic effect. Its effects are due to the inhibitory actions on COXs, which are involved in the synthesis of prostaglandins, agents that play an important role in the production of pain, inflammation, and fever. Because of high membrane permeability, the extent of IB absorption approaches up to 100%. Dissolution thus becomes the rate limiting step for absorption.

Specifically, the composition described herein comprising Zn(II)-Ibuprofen-complex can be formulated as oral solution or tablet. As an example, ibuprofen can be formulated as oral suspension wherein about 44.16 mg uncomplexed ibuprofen is present in combination with about 8.84 mg Zn(II)-Ibuprofen-complex. As a further example, ibuprofen can be formulated as 250 mg tablet wherein about 220.83 mg uncomplexed ibuprofen is present in combination with about 44.19 mg Zn(II)-Ibuprofen-complex. As a further example, ibuprofen can be formulated as 400 mg tablet wherein about 353.33 mg uncomplexed ibuprofen is present in combination with about 70.67 mg Zn(II)-Ibuprofen-complex. The Zn(II)-Ibuprofen-complex composition described herein may also be formulated as combination tablet, soft gel capsule, water soluble granules or effervescent tablet with vitamin C calcium ascorbate, caffein, calcium ascorbate ester C or chlorphenamine hydrogen maleate, wherein about 220.83 mg uncomplexed ibuprofen is contained in combination with about 44.19 mg Zn(II)-Ibuprofen-complex.

**Mefenamic Acid** (MA) is characterized by the structure 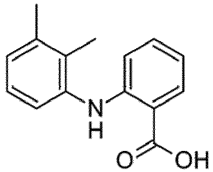 and is an anthranilic acid derivative. It is a member of the fenamate group of nonsteroidal anti-inflammatory drugs (NSAIDs). It exhibits anti-inflammatory, analgesic, and antipyretic activities. Similar to other NSAIDs, mefenamic acid inhibits prostaglandin synthetase. Mefenamic acid binds the prostaglandin synthetase receptors COX-1 and COX-2, inhibiting the action of prostaglandin synthetase. As these receptors have a role as a major mediator of inflammation and/or a role for prostanoid signaling in activity-dependent plasticity, the symptoms of pain are temporarily reduced. Anthranylic acid derivatives are direct structural analogs of salicylic acid derivatives. They possess analgesic, anti-pyretic and fever-reducing activity. They are similar to pyrazolones in terms of analgesic and fever-reducing activity, yet they exceed the anti-inflammatory activity of salicylates.

Specifically, the composition described herein comprising Zn(II)-Mefenamic Acid-complex can be formulated as ointment, solution for topical administration, vaginal suppository or ear drops. As an example, mefenamic acid can be formulated as oral suspension wherein about 8.22 mg uncomplexed mefenamic acid is present in combination with about 2.48 mg Zn(II)-Mefenamic Acid-complex in a 1 ml suspension. Alternatively, mefenamic acid can be formulated as tablet of 125 mg wherein about 102.72 mg uncomplexed mefenamic acid is contained in combination with about 31.03 mg Zn(II)-Mefenamic Acid-complex. Alternatively, mefenamic acid can be formulated as tablet of 250 mg wherein about 205.44 mg uncomplexed mefenamic acid is contained in combination with about 62.06 mg Zn(II)-Mefenamic Acid-complex. The Zn(II)-Mefenamic Acid-complex composition described herein may also be formulated as combination tablet, soft gel capsule, effervescent tablet or water soluble granules with vitamin C calcium ascorbate, caffein, calcium ascorbate ester C or chlorphenamine hydrogen maleate, wherein about 102.72 mg uncomplexed mefenamic acid is contained in combination with about 31.03 mg Zn(II)-Mefenamic Acid-complex.

**Diclofenac** is characterized by the structure 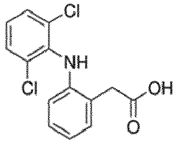 and inhibits cyclooxygenase-1 and -2, the enzymes responsible for production of prostaglandin (PG) G2 which is the precursor to other PGs. These molecules have broad activity in pain and inflammation and the inhibition of their production is the common mechanism linking each effect of diclofenac. PGE2 is the primary PG involved in modulation of nociception. It mediates peripheral sensitization through a variety of effects. PGE2 activates the Gq-coupled EP1 receptor leading to increased activity of the inositol trisphosphate/phospholipase C pathway. Activation of this pathway releases intracellular stores of calcium which directly reduces action potential threshold and activates protein kinase C (PKC) which contributes to several indirect mechanisms. PGE2 also activates the EP4 receptor, coupled to Gs, which activates the adenylyl cyclase/protein kinase A (AC/PKA) signaling pathway. PKA and PKC both contribute to the potentiation of transient receptor potential cation channel subfamily V member 1 (TRPV1) potentiation, which increases sensitivity to heat stimuli. They also activate tetrodotoxin-resistant sodium channels and inhibit inward potassium currents. PKA further contributes to the activation of the P2X3 purine receptor and sensitization of T-type calcium channels. The activation and sensitization of depolarizing ion channels and inhibition of inward potassium currents serve to reduce the intensity of stimulus necessary to generate action potentials in nociceptive sensory afferents. PGE2 acts via EP3 to increase sensitivity to bradykinin and via EP2 to further increase heat sensitivity. Central sensitization occurs in the dorsal horn of the spinal cord and is mediated by the EP2 receptor which couples to Gs. Pre-synaptically, this receptor increases the release of pro-nociceptive neurotransmitters glutamate, CGRP, and substance P. Post-synaptically it increases the activity of AMPA and NMDA receptors and produces inhibition of inhibitory glycinergic neurons. Together these lead to a reduced threshold of activating, allowing low intensity stimuli to generate pain signals. PGI2 is known to play a role via its Gs-coupled IP receptor although the magnitude of its contribution varies. It has been proposed to be of greater importance in painful inflammatory conditions such as arthritis. By limiting sensitization, both peripheral and central, via these pathways NSAIDs can effectively reduce inflammatory pain. PGI2 and PGE2 contribute to acute inflammation via their IP and EP2 receptors. Similar to β adrenergic receptors these are Gs-coupled and mediate vasodilation through the AC/PKA pathway. PGE2 also contributes by increasing leukocyte adhesion to the endothelium and attracts the cells to the site of injury. PGD2 plays a role in the activation of endothelial cell release of cytokines through its DP1 receptor. PGI2 and PGE2 modulate T-helper cell activation and differentiation through IP, EP2, and EP4 receptors which is believed to be an important activity in the pathology of arthritic conditions. By limiting the production of these PGs at the site of injury, NSAIDs can reduce inflammation. PGE2 can cross the blood-brain barrier and act on excitatory Gq EP3 receptors on thermoregulatory neurons in the hypothalamus. This activation triggers an increase in heat-generation and a reduction in heat-loss to produce a fever.

Specifically, the composition described herein comprising Zn(II)-Diclofenac-complex can be formulated as ointment, solution for topical administration, vaginal suppository or ear drops. As an example, diclofenac can be formulated as softgel capsule about 30,75 mg Zn(II)-Diclofenac-complex, transdermal patch (about 172,2 mg or 110,7 mg Zn(II)-Diclofenac-complex), emulsion (about 30.75 mg Zn(II)-Diclofenac-complex), eye drops (about 1.23 mg Zn(II)-Diclofenac-complex), ear drops (about 1.23 mg Zn(II)-diclofenac-complex).

**Acetylsalicylic Acid (ASA)/ Aspirin** is characterized by the structure 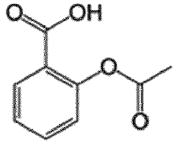 and disrupts the production of prostaglandins throughout the body by targeting cyclooxygenase-1 (COX-1) and cyclooxygenase-2 (COX-2). Prostaglandins are potent, irritating substances that have been shown to cause headaches and pain upon injection into humans. Prostaglandins increase the sensitivity of pain receptors and substances such as histamine and bradykinin. Through the disruption of the production and prevention of release of prostaglandins in inflammation, this drug may stop their action at pain receptors, preventing symptoms of pain. ASA is considered an antipyretic agent because of its ability to interfere with the production of brain prostaglandin E1. Prostaglandin E1 is known to be an extremely powerful fever-inducing agent. The inhibition of platelet aggregation by ASA occurs because of its interference with thromboxane A2 in platelets, caused by COX-1 inhibition. Thromboxane A2 is an important lipid responsible for platelet aggregation, which can lead to clot formation and future risk of heart attack or stroke. ASA blocks prostaglandin synthesis. It is non-selective for COX-1 and COX-2 enzymes. Inhibition of COX-1 results in the inhibition of platelet aggregation for about 7-10 days (average platelet lifespan). The acetyl group of acetylsalicylic acid binds with a serine residue of the cyclooxygenase-1 (COX-1) enzyme, leading to irreversible inhibition. This prevents the production of pain-causing prostaglandins. This process also stops the conversion of arachidonic acid to thromboxane A2 (TXA2), which is a potent inducer of platelet aggregation. Platelet aggregation can result in clots and harmful venous and arterial thromboembolism, leading to conditions such as pulmonary embolism and stroke. It is important to note that there is 60% homology between the protein structures of COX-1 and COX-2. ASA binds to serine 516 residue on the active site of COX-2 in the same fashion as its binding to the serine 530 residue located on the active site of COX-1. The active site of COX-2 is, however, slightly larger than the active site of COX-1, so that arachidonic acid (which later becomes prostaglandins) manages to bypass the aspirin molecule inactivating COX-2 ASA, therefore, exerts more action on the COX-1 receptor rather than on the COX-2 receptor. A higher dose of acetylsalicylic acid is required for COX-2 inhibition.

Specifically, the composition described herein comprising Zn(II)-Aspirin-complex can be formulated as water soluble granules, effervescent tablet or capsule. As an example, Zn(II)-Aspirin-complex can be formulated as water soluble granules with about 42.58 mg Zn(II)-Aspirin-complex and 469.1 mg uncomplexed Aspirin. The Zn(II)-Aspirin-complex composition described herein may also be formulated as combination tablet, soft gel capsule, effervescent tablet or water soluble granules with vitamin C calcium ascorbate, caffein, calcium ascorbate ester C or chlorphenamine hydrogen maleate, wherein about 375.27 mg uncomplexed aspirin is contained in combination with about 34.06 mg Zn(II)-Aspirin-complex.

**Dexibuprofen** is characterized by the structure

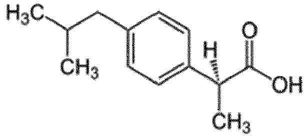

S(+)-ibuprofen, is a non-steroidal anti-inflammatory drug (NSAID). It is a pharmacologically effective enantiomer of racemic ibuprofen that differs in physicochemical properties. It is proposed to be more pharmacologically active and tolerable with a better safety profile than ibuprofen due to higher concentration of active S enantiomer. Dexibuprofen has a slower dissolution rate in the simulated gastric and enteric juices compared with the racemic ibuprofen and displays improved oral bioavailability. Like common NSAIDs, dexibuprofen is an active enantiomer of ibuprofen that suppresses the prostanoid synthesis in the inflammatory cells via inhibition of the COX-2 isoform of the arachidonic acid COX.

Specifically, the composition described herein comprising Zn(II)-Dexibuprofen-complex can be formulated as water soluble granules, effervescent tablet or capsule. As an example, Zn(II)-Dexibuprofen-complex can be formulated as water soluble granules with about 33.14 mg Zn(II)-Dexibuprofen-complex and 102.79 mg uncomplexed dexibuprofen. The Zn(II)-Dexibuprofen-complex composition described herein may also be formulated as combination tablet, soft gel capsule, effervescent tablet or water soluble granules with vitamin C calcium ascorbate, caffein, calcium ascorbate ester C or chlorphenamine hydrogen maleate, wherein about 164.47 mg uncomplexed dexibuprofen is contained in combination with about 53.03 mg Zn(II)-dexibuprofen-complex.

**Flurbiprofen** is characterized by the structure

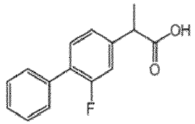

Flurbiprofen, a nonsteroidal anti-inflammatory agent (NSAIA) of the propionic acid class, is structurally and pharmacologically related to fenoprofen, ibuprofen, and ketoprofen, and has similar pharmacological actions to other prototypical NSAIAs. Flurbiprofen exhibits anti-inflammatory, analgesic, and antipyretic activities. The commercially available flurbiprofen is a racemic mixture of (+)S- and (-) R-enantiomers. The S-enantiomer appears to possess most of the anti-inflammatory, while both enantiomers may possess analgesic activity. Similar to other NSAIAs, the anti-inflammatory effect of flurbiprofen occurs via reversible inhibition of cyclooxygenase (COX), the enzyme responsible for the conversion of arachidonic acid to prostaglandin G2 (PGG2) and PGG2 to prostaglandin H2 (PGH2) in the prostaglandin synthesis pathway. This effectively decreases the concentration of prostaglandins involved in inflammation, pain, swelling and fever. Flurbiprofen is a non-selective COX inhibitor and inhibits the activity of both COX-1 and -2. It is also one of the most potent NSAIAs in terms of prostaglandin inhibitory activity.

Specifically, the composition described herein comprising Zn(II)-Flurbiprofen-complex can be formulated as oral spray, gargle solution, optionally with chlorhexidine; pastilles, eye drops, optionally with tyloxapol or povidone; or ointment, optionally with oytyldodecanol or dexpanthenol.

As an example, flurbiprofen can be formulated as lozenges, comprising about 8.75 mg Zn(II)- Flurbiprofen-complex. Alternatively, flurbiprofen can be formulated as oral spray/ mouth wash comprising about 3.2 mg Zn(II)-Flurbiprofen-complex), transdermal patch (51.2 mg Zn(II)-Flurbiprofen-complex), eye drops/ ear drops (about 0.154 mg Zn(II)-Flurbiprofen-complex), soft gel capsules (about 51.12 mg Zn(II)-Flurbiprofen-complex or 63.9 mg Zn(II)-Flurbiprofen-complex).

A **hydrophilic surfactant** as defined herein has an HLB (hydrophobic-lipophilic balance) of > 10. HLB is an empirical expression for the relationship of the hydrophilic and hydrophobic groups of a surface-active amphiphilic molecule, such as a surfactant). It is used to index surfactants and its value varies from about 1 to about 45. The higher the HLB, the more water-soluble the surfactant.

Herein, the hydrophilic surfactant is any one of PEG 300, PEG 400, PEG15, polyoxyethylene (20) cetyl ether, ethanol, propylene glycol, glycerol, and diethylene glycol monoethyl ether or combinations thereof.

Specifically, PEG 300, PEG 400, PEG15, polyoxyethylene (20) cetyl ether, ethanol, propylene glycol, glycerol, and diethylene glycol monoethyl ether are synthetically produced and are pure chemical compounds.

A mucoadhesive agent, when placed in the oral cavity in contact with the mucosa therein, adheres to the mucosa. The mucoadhesive agent is especially effective in transmucosal delivery of the Zn(II)-NSAID complex composition described herein., as the mucoadhesive agent permits a close and extended contact of the composition with the mucosal surface by promoting adherence of the composition or drug to the mucosa, and facilitates the release of the active ingredient, i.e. the NSAID from the composition. The mucoadhesive agent can be a polymeric compound, such as a cellulose derivative but it may be also a natural gum, alginate, pectin, or such similar polymer. The concentration of the mucoadhesive agent in the coating, such as a powder matrix coating, may be adjusted to vary the length of time that the film adheres to the mucosa or to vary the adhesive forces generated between the film and mucosa. The mucoadhesive agent may adhere to oral mucosa or to mucosa or tissue in other parts of the body, including the mouth, nose, eyes, vagina, and rectum.

Mucoadhesive agents suitable for use in the pharmaceutical composition of the invention include:
Natural polymers, specifically hyaluronate, carrageenan, pectin, alginate, xanthan gum, and guar gum, tragacanth gum, guar gum, karaya gum, carrageenan, xanthan gum, gellan gum, locust bean gum;
Cyclodextrins, specifically α cyclodextrin, β cyclodextrin, γ cyclodextrin, and methylated β-cyclodextrins such as RAMEB;
Hydrogen bond polymers, specifically acrylate, more specifically hydroxylated methacrylate poly(methacrylic acid), butylmethacrylate, poly[acrylic acid-co-(2-ethylhexyl)acrylate-co-methylacrylate], high molecular weight crosslinked polyacrylic acid polymers (Carbopols^{™} (CP)), and polycarbophil (PC),
Cationic compounds, specifically poly-I-arginine and l-lysine;
Non-ionic polymers, specifically hydroxyethyl starch, hydroxyethylmethacrylate copolymers, hydroxypropyl cellulose (HPC), methyl cellulose, hydroxypropyl methyl cellulose, poly(ethylene oxide), polyvinyl alcohol (PVA), polyvinylpyrrolidone (PVP), scleroglucan, polyoxyethylene poly (methacrylic acid co-methyl methacrylate), carboxyvinyl copolymers, methyl methacrylate copolymers, ethylene vinyl acetate, dimenthylpolysiloxanes, polyoxyalkylene block copolymers; and
Positively charged polymers, specifically chitosan, trimethylchitosan, aminodextran, dimethylaminoethyl (DMAE), diethylaminoethyl (DEAE), dextran.

In accordance with the present invention, the pharmaceutical composition comprises two molecules of acetylsalicylic acid, flurbiprofen, diclofenac, mefenamic acid, dexibuprofen, or ibuprofen that reacted with one molecule of zinc in the presence of water.

Specifically, the zinc salt is chloride, however, according to alternative embodiments, the salt can be zinc acetate, and zinc chloride.

In general, NSAIDs have several side effects. Some of the cytoprotective prostaglandins formed via oxygenation of arachidonic acid are essential for the smooth functioning of the gastrointestinal tract. Regular uptake of NSAIDs induces gastric or intestinal ulceration by damaging the gastroduodenal mucosa.

Inhibition of COX-1 results in GI tract toxicity along with reduced prostaglandin formation, whereas COX-2 selective NSAIDs (COXIBs) exhibit excellent anti-inflammatory activity without damaging the GI tract.

Several Zinc(II)-NSAID complexes supported by NO-donating 1,10-phenanthrolinefuroxan exhibit anti-inflammatory activities through selective inhibition of the COX-2 pathway. The strategy represents a general procedure to convert non-selective or COX-1 selective NSAIDs to selective COX-2 inhibitors. It has been observed that nitric oxide (NO) has similar cytoprotective activities as PGs on gastric mucosa, and also plays important roles in vasodilation, inhibition of platelet aggregation and smooth muscle cell proliferation. Thus, coupling a NO-donating moiety to an NSAID is expected to deliver NO to the site prone to damage, thereby decreasing gastric toxicity and reducing cardiotoxicity.

The NO-NSAIDs (e.g. NO-Aspirin, NO-ibuprofen, NO-indomethacin, NO-Flurbiprofen, NO-Dexibuprofen) that have been testes so far, are organic compounds in which the NO donating groups are covalently attached to conventional NSAID by an aromatic or aliphatic spacer.

To retain the anti-inflammatory activity upon functionalization of NO-NSAIDs, ternary zinc(II)-NSAID complexes of the NO-donating chelating 'furoxan' ligand have been developed. In the ternary metal complexes, the metal centers act as spacers.

The research is focused on combinations of 1,10-phenanthroline-derived furoxan for coordination with metal ions, and different NSAIDs that are known to bind with metal ions through carboxylate oxygen donors. A number of metal-NSAID complexes have been reported to exhibit anti-inflammatory activities. Combining the NO-donor group and the NSAID in a single molecule will overcome major problems of introducing different drug molecules individually to the target of interest. Additionally, the enhanced efficacy and selective COX inhibitory properties of NSAID may be brought about through coordination of both NO-donors and NSAIDs to a metal center.

Furoxans (1,2,5-oxadiazole-2-oxides) are well known heterocycles capable of donating nitric oxide in the presence of excess l-cysteine. Additionally, 4,4'-Bipyridine, 2,9-Dimethyl-Phenanthrolin, 1,2-Dimethylimidazol are also being tested as bioactive ligands.

The Zn(II)-dichloro complex of 1,10-phenanthroline-furoxan is synthesized from the reaction of equimolar amounts of 1,10-phenanthroline-derived furoxan and ZnCl2 in methanol. The ternary metal complexes are synthesized by mixing the precursor Zn(II)-dichloro complex with two equivalents of the respective sodium salt of NSAID.

Specifically, the Zn(II)-NO-NSAID complex is provided as the pure chemical compound, e.g. synthetically produced from synthesis of zinc chloride and 1,10-phenanthroline-derived furoxan with flurbiprofen (2-(3-fluoro-4-phenylphenyl)propanoic acid), zinc chloride and diclofenac (2-[2-(2,6-dichloroanilino)phenyl]acetic acid), zinc chloride and aspirin (acetylsalicylic acid), zinc chloride and mefenamic acid (2-(2,3-dimethylanilino)benzoic acid), zinc chloride and dexibuprofen ((2S)-2-[4-(2-methylpropyl)phenyl]propanoic acid), and zinc chloride and ibuprofen (2-[4-(2-methylpropyl)phenyl]propanoic acid).

The Zn(II)-NSAID complex is synthesized from the reaction of equimolar amounts of two equivalents of the respective sodium salt of an NSAID (acetylsalicylic acid, flurbiprofen, diclofenac, mefenamic acid, dexibuprofen, or ibuprofen) and ZnCl2 in acetone. The solution is being stirred for 3 h then evaporated to dryness to get the solid residue. The solid product was then washed with ether to remove any excess reactants and allowed to dry in air.

Specifically, the Zn(II)-NSAID complex is provided as the pure chemical compound, e.g. synthetically produced from synthesis of zinc chloride and flurbiprofen (2-(3-fluoro-4-phenylphenyl)propanoic acid), zinc chloride and diclofenac (2-[2-(2,6-dichloroanilino)phenyl]acetic acid), zinc chloride and aspirin (acetylsalicylic acid), zinc chloride and mefenamic acid (2-(2,3-dimethylanilino)benzoic acid), zinc chloride and dexibuprofen ((2S)-2-[4-(2-methylpropyl)phenyl]propanoic acid), and zinc chloride and ibuprofen (2-[4-(2-methylpropyl)phenyl]propanoic acid).

According to a specific embodiment, the maximum daily dose, the assumed average maintenance dose per day ("DDD") for a drug used for its main indication in adults, of the pharmaceutical composition is assumed as follows: for Zn(II)-ibuprofen it is about 212.12 mg, for Zn(II)-mefenamic it is about 248.22 mg; for Zn(II)-aspirin it is about 255.47 mg; for Zn(II)-flurbiprofen it is about 255.6 mg; for Zn(II)-diclofenac it is about 123 mg (buccal application) to about 226 mg (topical application); and for Zn(II)-dexibuprofen it is about 212.12 mg.

According to a further specific embodiment, the composition provided herein comprises, in addition to the Zn(II)-NSAID-complex and/or Zn(II)-NO-NSAID-complex compound, further comprises uncomplexed NSAID, specifically ibuprofen, ibuprofen lysinate, ibuprofen arginate; ibuprofen sodium salt, ibuprofen calcium salt; mefenamic acid, sodium mephenamate, mefenamin-lysinate, mefenamin arginate; dexibuprofen, dexibuprofen lysine; aspirin, aspirin calcium, and aspirin sodium salt until the assumed average maintenance dose per day ("DDD") for a drug used for its main indication in adults, as listed in the ATC index, is reached.

Specifically, the pharmaceutical composition as described herein comprises a DDD combination amount of about 890 mg dexibuprofen and about 310 mg Zn(II)-dexibuprofen; a DDD-combination of about 890 mg ibuprofen and about 310 mg Zn(II)-ibuprofen; a DDD-combination of about 1000 mg-1200 mg ibuprofen lysine/ ibuprofen arginate/ ibuprofen sodium salt/ ibuprofen calcium salt and about 310 mg Zn(II)-ibuprofen; a DDD-combination of about 2650 mg-3000 mg of aspirin/aspirin calcium, and aspirin sodium salt and 358 mg Zn(II)-aspirin; a DDD combination of about 650 mg mefenamic acid/ and about 350 mg Zn(II)-mefenamin; a DDD-combination of about 850 mg-1100 mg sodium mefenamate/ mefenamin lysinate/ mefenamin arginate and about 350 mg Zn(II)-mefenamin.

Specifically, the uncomplexed NSAIDs as listed herein are pure chemical compounds, specifically they are synthetically produced.

The pharmaceutical composition can be combined with further agents or supplements such as, but not limited to vitamins, trace minerals, calcium ascorbate, caffeine, caffeine citrate, chlorpheniramine maleate, D-pseudoephedrine, or pseudoephedrine sulfate.

Caffeine is both a xanthine alkaloid and a stimulant. Acting on the central nervous system, caffeine helps ward off drowsiness and increases alertness. It is an additive found in many foods and medications.

Chlorpheniramine maleate, D-pseudoephedrine, or pseudoephedrine sulfate are antihistamines. Further examples of antihistamines include diphenhydramine hydrochloride, diphenhydramine salicylate, diphenhydramine, chlorpheniramine hydrochloride, isothipendyl hydrochloride, tripelennamine hydrochloride, promethazine hydrochloride, methdilazine hydrochloride, cetirizine, brompheniramine, clemastine, fexofenadine, loratadine, etc.

Said compounds are provided specifically as pure chemical compounds, specifically they are synthetically produced.

Vitamins include vitamins A, B, C, D, E, and K. In some implementations, trace minerals may be added to the pharmaceutical composition, such as calcium, iron, phosphorous, iodine, magnesium, zinc, selenium, copper, manganese, chromium, molybdenum, chloride, potassium, or any combination of the foregoing.

Specifically, the pharmaceutical preparation of the Zn(II)-NSAID-complex as described herein, optionally comprising NSAIDs and its compounds, further optionally comprises about 50-75mg of calcium ascorbate, or about 25 to 50mg of caffeine/ caffeine citrate, or about 1,5-5mg of D-pseudoephedrine/ pseudoephedrine sulfate, and any combination thereof.

The pharmaceutical composition can comprise one or more pharmaceutically acceptable excipients or carriers.

As used herein, a **"pharmaceutically acceptable carrier"** refers to a carrier or diluent that does not cause significant irritation to an organism and does not abrogate the biological activity and properties of the administered pharmaceutical composition. It includes, but is not limited to, a diluent, excipient, vehicle, buffer, stabilizer, or preservative, and the like with which the pharmaceutical composition is administered. Pharmaceutically acceptable carriers generally include any and all suitable solvents, dispersion media, coatings, antiviral, antibacterial and antifungal agents, isotonic and absorption delaying agents, and the like that are physiologically compatible with the pharmaceutical composition described herein. Saline solutions and aqueous dextrose and glycerol solutions can also be employed as liquid carriers. Suitable excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E.W. Martin. The formulation should be selected according to the mode of administration.

Tablets can contain excipients, glidants, fillers, binders, disintegrants, lubricants, flavors and the like. It is furthermore preferred to provide for a preparation formulated to act at the site of the mucosa, e.g. at mucosal sites (such as nose, mouth, eyes, esophagus, throat, lung), e.g. locally without systemic action. Aqueous formulations are prepared in sterile form.

Herein provided is also the pharmaceutical composition described herein in the form of an emulsion, such as a water in oil (w/o), an oil in water (o/w) or a bicontinuous system emulsion, further comprising a co-surfactant, an oil, and an aqueous solution.

In a bicontinuous structure the water phase and the oil phase are each three-dimensionally continuous. Specifically, bicontinuous system emulsions are dynamic, isotropic, thermodynamically stable, and optically clear systems consisting of water and oil nanochannels separated by flexible surfactant monolayers of near zero-curvature, are a potentially valuable biomembrane mimetic system that resemble oil-swollen biomembranes.

The structure of emulsions (water/oil, oil/water or bicontinuous) can be determined by means of published methods e.g. NMR, electron microscope, or electrical conductivity (Shinoda K. and Uchimura T., 2018, Evaluating the Creaming of an Emulsion via Mass Spectrometry and UV-Vis Spectrophotometry., ACS Omega, 22:3(10) 13752-13756; Shinoda K. et al., 1991, Lecithin-based microemulsions: phase behavior and microstructure, J Phys Chem, 95(2), 989-993).

The emulsion can comprise droplets, specifically with a droplet size in the range of 10 to 1000 nm, 10 to 900 nm, 10 to 800, 10 to 700 nm, 10 to 600 nm, 10 to 500 nm, specifically of 10 to 500 nm, 10 to 400 nm, 10 to 300 nm, specifically of 10 to 200 nm, specifically of 10 to 150 nm.

According to a specific embodiment, the droplet size of said emulsion is in the range of 100 to 500 nm for nanoemulsion, specifically 10 to 150 nm for microemulsion, specifically less than 100 nm for self-microemulsifying drug delivery system (SMEDDS), and specifically 20 to 200 nm for self-nanoemulsifying drug delivery system (SNEDDS).

Specifically, SNEDDS are defined as isotropic mixtures of natural or synthetic oils, solid or liquid surfactants or, alternatively, one or more hydrophilic solvents and cosolvents/surfactants that have the ability of forming fine oil-in-water (o/w) microemulsions upon mild agitation followed by dilution in aqueous media such as gastric fluids. SNEDDS spread readily in the gastrointestinal tract, and the digestive motility of the stomach and the intestine provide the agitation necessary for self-emulsification. SNEDDS is defined as a stable mixture of drug, oil, surfactant, and co-surfactant.

Specifically, SMEDDS is defined as self-emulsifying drug delivery system which can form fine oil-in-water droplets with a diameter size of less than 50 nm under mild agitation of the gastrointestinal tract without the dissolution process. In addition, SMEDDS has the potential to deliver poorly water-soluble drugs because the microemulsion droplets of SMEDDS provide 100% of the capacity to entrap a drug and protects the drug from gastrointestinal degradation. The increased surface area and small droplets that results in transporting the drug substance through the unstirred water layer to the gastrointestinal membrane contribute to a significant increase in drug absorption. Moreover, the droplets can be rapidly dispersed in blood as well as lymph, and the lymphatic drug transport can avoid the first-pass effect.

The oil phase can be any one of vegetable oils (e.g. corn oil, olive oil, peanut oil, rapeseed oil, sesame oil, soybean oil, coconut or palm seed oil), hydrogenated vegetable oils (e.g., Lubritab^{®}, AkofineTM, Sterotex^{®}, Dynasan^{®} P60, Softisan^{®} 154, Suppocire^{®}, Cutina^{®} HR, Sterotex^{®} HM, Hydrocote, Lipo), or partial glycerides (e.g. Capmul^{®} MCM, Geleol^{®}, Imwitor^{®} 191, Cutina^{®} GMS, Tegin^{®}, Precirol^{®} ATO 5, Peceol^{®}, Maisine^{®} 35-1, Compritol^{®} 888 ATO).

The co-surfactant can be nonionic surfactant that has been reported as safe for oral administration in concentrations relevant for stabilization, such as but not limited to polyoxylglycerides (e.g. Labrasol^{®}, Labrafil^{®}, Gelucire^{®}), ethoxylated glycerides derived from castor oil (e.g. Cremophor EL, RH40, or RH60), esters of fatty acids and alcohols (e.g., Plurol^{®} Oleique CC497, Capryol^{®} 90, Lauroglycol^{™} 90, Mirj^{®} 45, Mirj^{®} 52, Solutol^{®} HS15, Span^{®} 80, Span^{®} 20, Tween^{®} 80, Tween^{®} 20) or sucrose ester-based natural surfactants (e.g. sucrose laurate L-1695, sucrose stearate S-970, sucrose monolaurate + sucrose dilaurate, sucrose laurate D-1214, sucrose myristate C-1416, sucrose palmitate P-1670 -/+ sucrose stearate S-970). Non-ionic surfactants neither form cations nor anions in water. Their solubility in water is based on the binding of the hydrophilic parts to the water molecules.

In addition, the emulsion may comprise dexpanthenol, which is an alcohol derivative of pantothenic acid, a component of the B complex vitamins. Preferably, dexpanthenol is a pure chemical compound, e.g. synthetically produced.

Specifically, the pharmaceutical composition or emulsion provided herein, comprises 0,25%, 0,5%, 1%, 1.5%, 2%, 2.5%, 3%, 3.5%, 4%, 4.5% or 5% of Zn(II)-Ibuprofen-complex.

Specifically, the pharmaceutical composition or emulsion provided herein, comprises 0,25%, 0,5%, 0,75%, 1%, 1.5%, 2%, 2.5%, 3%, 3.5%, 4%, 4.5% or 5% of Zn(II)-Mefenamin-complex.

Specifically, the pharmaceutical composition or emulsion provided herein comprises 1.028 mg, 1.1 mg, 1.2 mg, 12 mg, 12.5 mg, 13 mg, 14 mg, 14,27 mg, 25 mg, 25,5 mg, 26 mg, 26.5 mg, 27 mg, 27.5 mg, 28 mg, 28.5 mg, 29 mg, 29.5 mg, 30 mg, 30.5 mg, 31 mg, 31.5 mg, 32 mg, 100 mg, 105 mg, 110 mg, 111 mg, 112 mg, 113 mg, 114 mg, 115 mg, 140 mg, 145 mg, 150 mg, 155 mg, 160 mg, 165 mg, 170 mg or 175 mg of Zn(II)-Diclofenac-complex.

Specifically, the pharmaceutical composition or emulsion provided herein comprises 0.12 mg, 0.13 mg, 0.14 mg, 0.145 mg, 0.15 mg, 0.155 mg, 0.16 mg, 0.165 mg, 2.5 mg, 2.6 mg, 2.7 mg, 2.8 mg, 2.9 mg, 3 mg, 3,1 mg, 3.2 mg, 3.3 mg, 3.4 mg, 3.5 mg, 8.7 mg, 8.8 mg, 8.9 mg, 9 mg, 9.5 mg, 10 mg, 10,5 mg, 11 mg, 11.1 mg, 11.2 mg, 11.3 mg, 11.4 mg, 11.5 mg, 12 mg, 12.5 mg, 12.8 mg, 13 mg, 20 mg, 21 mg, 22 mg, 23mg, 24mg, 2 5mg, 26 mg, 27 mg, 28 mg, 29 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg or 55 mg of Zn(II)-Flurbiprofen-complex.

Co-surfactants can be nonionic tensides, specifically polyoxylglycerides (e.g. Labrasol^{®}, Labrafil^{®}, Gelucire^{®}), ethoxylated glycerides derived from castor oil (e.g. Cremophor EL, RH40, or RH60), esters of fatty acids and alcohols (e.g., Plurol^{®} Oleique CC497, Capryol^{®} 90, Lauroglycol^{™} 90, Mirj^{®} 45, Mirj^{®} 52, Solutol^{®} HS15, Span^{®} 80, Span^{®} 20, Tween^{®} 80, Tween^{®} 20) or sucrose ester-based natural surfactants (e.g. sucrose laurate L-1695, sucrose stearate S-970, sucrose monolaurate + sucrose dilaurate, sucrose laurate D-1214, sucrose myristate C-1416, sucrose palmitate P-1670 -/+ sucrose stearate S-970).

Further provided herein is a drug-in-mucoadhesive transdermal patch comprising a backing layer selected from the group consisting of any one of plastic or polymer, and a combination thereof,
and an adhesive layer comprising
a) a Zn-NSAID complex with flurbiprofen in one or more salt forms,
b) polyethylene glycol, specifically PEG400, and
c) a combination of mucoadhesive agents, specifically poly(N-2-vinylpyrrolidone) (PVP) microspheres, and/or polyisobutylene, and/or acrylate, and/or silicone.

Further encompassed herein is also a drug-in-mucoadhesive transdermal patch comprising a backing layer selected from the group consisting of any one of plastic or polymer and a combination thereof,
and a mucoadhesive layer comprising
a. a Zn (II)-NSAID complex with diclofenac in one or more salt forms, and
b. a combination of mucoadhesive agents, specifically butylmethacrylate-copolymer, poly[acrylic acid-co-(2-ethylhexyl)acrylate-co-methylacrylate], and/or polyisobutylene, and/or acrylate, and/or silicone.

In order to secure the additional anti-viral and anti-bacterial effects, the pharmaceutical composition comprising the Zn (II)-NSAID-complexes allows in vivo delivery at respective physiological pH-values, i.e. oral and pulmonary delivery at pH 7.4, nasal delivery at pH 5.3-5.6, for topical delivery at pH 5.0-7.4, for buccal delivery pH 6-6.8.

### EXAMPLES

The examples described herein are illustrative of the present invention and are not intended to be limitations thereon. Many modifications and variations may be made to the techniques described and illustrated herein without departing from the spirit and scope of the invention. Accordingly, it should be understood that the examples are illustrative only and are not limiting upon the scope of the invention.

### Example 1: Virtual bioequivalence and clinically relevant specifications using in vitro biorelevant in vitro solubility experiments

Physiologically-based population pharmacokinetic modeling (popPBPK) coupled with in vitro biopharmaceutics tools such as biorelevant dissolution testing can serve as a powerful tool to establish virtual bioequivalence and set clinically relevant specifications. One of several applications of popPBPK modeling is in the emerging field of virtual bioequivalence (VBE), where it can be used to streamline drug development by implementing model-informed formulation design and to inform regulatory decision-making e.g., with respect to evaluating the possibility of extending BCS-based biowaivers beyond BCS Class I and III compounds. A new pharmacokinetic modeling with Naproxen as model compound was developed recently (Loisios-Konstantinidis I. et. al., 2020, Establishing virtual bioequivalence and clinically relevant specifications using in vitro biorelevant dissolution testing and physiologically-based population pharmacokinetic modeling. case example: Naproxen, European Journal of Pharmaceutical Sciences, 143:105170) and found suitable for the following experiments on the in vitro solubility and dissolution of Zn(II)-NSAID complexes.

The following Zn(II)-NSAID complexes are investigated:
- Zn(II)-Mefenamin
- Zn(II)-Ibuprofen
- Zn(II)-Flurbiprofen
- Zn(II)-Diclofenac
- Zn(II)-Aspirin
- Zn(II)-Dexibuprofen
- Zn(II)-NO-Ibuprofen
- Zn(II)-NO-Flurbiprofen
- Zn(II)-NO-Dexibuprofen

The solubility of Zn(II)-NSAID complexes and Zn(II)-NO-NSAID-complexes is investigated in various selected aqueous and biorelevant dissolution media using the Uniprep^{™} system (Whatman^{®}, Piscataway, NJ, USA). All aqueous buffers are prepared according to the European Pharmacopoeia, while the biorelevant media are prepared according to Markopoulos et al. (2015, Eur J Pharm Biopharm., 93:173-82) and Fuchs et al. (2015, European Journal of Pharmaceutics and Biopharmaceutics, 94:229-240). The composition and physicochemical characteristics of the fasted and fed state biorelevant media used are summarized in Table 1.

An excess amount of API is added to 3 mL of dissolution medium and the samples are incubated for 24 h at 37°C on an orbital mixer. The samples are then filtered through the 0.45 µm PTFE filter integrated in the Uniprep^{™} system. The filtrate is immediately diluted with mobile phase and analyzed by high-performance liquid chromatography (HPLC). All measurements are performed at least in duplicate (n≥2).

An experimental estimate of the Zn(II)-NSAID-complex pKa is obtained by fitting the Henderson-Hasselbalch equation to the mean aqueous equilibrium solubility values using the SIVA Toolkit^{®} (n=6; all aqueous buffers). As intrinsic solubility the lowest reported value in buffers is used. The pKa is then compared with values available in the literature to confirm the validity of the aqueous solubility parameter estimates. The impact of bile salt concentration and subsequent formation of micelles on the solubility of Zn(II)-NSAID-complex is investigated. This is done by mechanistically modelling the mean solubility values in fasted state biorelevant media (n=3), accounting also for the relative proportions of Zn(II)-NSAID-complex solubilized in the aqueous versus the micelle phases, using the total solubility equation in SIVA Toolkit^{®} version 3.0 (SIVA; Certara, Simcyp Division; Sheffield, UK). Estimates of the logarithm of the micelle-water partition coefficient for the neutral and ionized drug are obtained to quantify the micelle-mediated solubility. Estimation of the relevant parameters is performed using the Nelder-Mead algorithm and weighting by the reciprocal of the predicted values is chosen. After model verification, all obtained estimates are used as input parameters for the development of the physiologically-based pharmacokinetic model (PBPK) model.

**Table 1: Physicochemical characteristics of the fasted and fed state biorelevant media**

| | **Fasted State** | | | | | | **Fed State** | | |
|---|---|---|---|---|---|---|---|---|---|
| | FaSS G F Level I | FaSS G F Level III | FaSSI F Level II | FaSSI F V3 Level I | FaSSI F V3 Level II | FeSSG F_{middle} Level II | FeSSI F Level I | FeSSI F Level II | FeSSI F V2 Level II |
| Sodium | - | 0.08 | 3.0 | - | 1.4 | - | - | 15 | 10 |
| Tauroch olate (mM) | | | | | | | | | |
| Glycoch olate (mM) | - | - | - | - | 1.4 | - | - | - | - |
| Glyceryl monoole ate (mM) | - | - | - | - | - | - | - | - | 5 |
| Sodium Oleate (mM) | - | - | - | - | 0.315 | - | - | - | 0.8 |
| Lecithin (mM) | - | 0.02 | 0.75 | - | 0.035 | - | - | 3.75 | 2 |
| Lysolecit hin (mM) | - | - | - | - | 0.315 | - | - | - | - |
| Choleste rol (mM) | - | - | - | - | 0.2 | - | - | - | - |
| Pepsin (mg/mL) | - | 0.1 | - | - | - | - | - | - | - |
| Sodium dihydrog en phospha te (mM) | - | - | 28.7 | 13.51 | 13.51 | - | - | - | - |
| NaOH (mM) | - | - | 13.8 | 3.19 | 3.19 | - | 101 | 101 | 102.4 |
| Acetic acid (mM) | - | - | - | - | - | 18.31 | 144 | 144 | - |
| Maleic acid (mM) | - | - | - | - | - | - | - | - | 71.9 |
| Sodium acetate (mM) | - | - | - | - | - | 32.98 | - | - | - |
| Lipofund in^{®}: buffer | - | - | - | - | - | 8.75: 91.25 | - | - | - |
| Hydrochl oric acid | q.s. pH 1.6 | q.s. pH 1.6 | - | - | - | q.s. pH 5 | - | - | - |
| Sodium chloride (mM) | - | 34.2 | 106 | - | 91.62 | 181.7 | - | 204 | 125.5 |
| Osmolali ty (mOsm/ kg) | - | 121 | 270 | - | 215 | 400 | - | 635 | 390 |
| Buffer capacity (HCI)((m mol/L)/Δ pH) | n.a. | n.a. | 12 | 5.6 | 5.6 | 25 | 76 | 76 | 25 |
| pH | 1.6 | 1.6 | 6.5 | 6.7 | 6.7 | 5.0 | 5.0 | 5.0 | 5.8 |

### Example 2: Virtual bioequivalence and clinically relevant specifications using in vitro biorelevant dissolution testing

Physiologically-based population pharmacokinetic modeling (popPBPK) coupled with in vitro biopharmaceutics tools such as biorelevant dissolution testing can serve as a powerful tool to establish virtual bioequivalence and set clinically relevant specifications. One of several applications of popPBPK modeling is in the emerging field of virtual bioequivalence (VBE), where it can be used to streamline drug development by implementing model-informed formulation design and to inform regulatory decision-making e.g., with respect to evaluating the possibility of extending BCS-based biowaivers beyond BCS Class I and III compounds. A new pharmacokinetic modeling with Naproxen as model compound was developed recently (Loisios-Konstantinidis I. et. al., 2020, Establishing virtual bioequivalence and clinically relevant specifications using in vitro biorelevant dissolution testing and physiologically-based population pharmacokinetic modeling. case example: Naproxen, European Journal of Pharmaceutical Sciences, 143:105170) and found suitable for the following experiments.

The following Zn(II)-NSAID complexes are investigated:
- Zn(II)-Mefenamin softgel capsule
- Zn(II)-Mefenamin water soluble powder
- Zn(II)-Ibuprofen softgel capsule
- Zn(II)-Ibuprofen water soluble powder
- Zn(II)-Flurbiprofen lozenge
- Zn(II)-Diclofenac softgel capsule
- Zn(II)-Aspirin water soluble powder
- Zn(II)-Dexibuprofen softgel capsule
- Zn(II)-NO-Ibuprofenwater soluble powder
- Zn(II)-NO-Ibuprofen softgel capsule
- Zn(II)-NO-Flurbiprofen lozenge

All dissolution tests are performed using calibrated USP II (paddle) apparatus (Erweka DT 80, Heusenstamm, Germany) at 37±0.4°C. Each vessel contained 500 mL of fresh, pre-warmed medium and the rotational speed is set at 75 rpm. Samples are withdrawn at 2.5, 5, 10, 15, 20, 30, 45, 60, 90 and 120 minutes via a 5 mL glass syringe connected to a stainless-steel cannula containing a 10 µm polyethylene cannula filter. Immediately thereafter, the sample is filtered through a 0.45 µm PTFE filter (ReZist^{™} 30, GE Healthcare UK Ltd., Buckinghamshire, UK), discarding the first 2 mL. The filtrate is immediately diluted with mobile phase and analyzed by HPLC-UV. The removal of 5 mL at each sampling time is taken into account in the calculation of the percentage dissolved. All experiments are performed at least in duplicate (n≥2) and the final pH in the vessel is recorded.

Since the conventional one-stage USP II dissolution test does not include a gastric compartment to account for disintegration of the dosage form in the stomach, differences in the disintegration time might bias the interpretation of the biorelevant in vitro dissolution behavior with respect to the in vivo performance. Therefore, to investigate the disintegration effect on the in vitro performance of the new formulations, a two-stage dissolution test for FaSSIF V3 is developed based on Mann et al. (2017, Validation of Dissolution Testing with Biorelevant Media: An OrBiTo Study., Mol Pharm., 14(12):4192-4201).

The dosage form is initially exposed to 250 mL of FaSSGF Level III and samples are removed at 5, 10, 15, 20, 30 minutes and via a 5 mL glass syringe connected to a stainless-steel cannula containing a 10 µm polyethylene cannula filter. Immediately thereafter, the sample is filtered through a 0.45 µm PTFE filter (ReZist^{™} 30, GE Healthcare UK Ltd., Buckinghamshire, UK), discarding the first 2 mL. The filtrate is immediately diluted with mobile phase and analyzed by HPLC-UV. The removal of 5 mL at each sampling time is taken into account in the calculation of the percentage dissolved. All experiments are performed at least in duplicate (n≥2) and the final pH in the vessel is recorded.

After the withdrawal of the last sample, 6.8 mL of 1 M sodium hydroxide and immediately thereafter 250 mL of FaSSIF V3 concentrate pH=6.7 (double concentration of all the constituents, apart from sodium hydroxide) are added to the vessel. Instead of increasing the pH of the intestinal medium concentrate to counterbalance the acidic pH of the stomach medium as described in the original study, (Mann et al., 2017, Validation of Dissolution Testing with Biorelevant Media: An OrBiTo Study., Mol Pharm., 14(12):4192-4201) sodium hydroxide is added first, but almost simultaneously, with the FaSSIF V3 concentrate. This is done to avoid using a very high pH in the FaSSIF V3 concentrate. After addition of sodium hydroxide and concentrated FaSSIF V3, further samples are removed at 32.5, 35, 40, 45, 50, 60 and 90 minutes.

The two-stage experiments are performed using calibrated USP II (paddle) apparatus (Erweka DT 80, Heusenstamm, Germany) at 37±0.4°C and the samples are analyzed by HPLC-UV. All experiments are performed at least in duplicate (n≥2) and the final pH in the vessel is recorded.

Samples obtained from solubility and dissolution experiments are first filtered through a 0.45 |jm PTFE filter (ReZist^{™} 30 syringe filter or Uniprep^{™}; Whatman^{®}, Piscataway, NJ, USA) and subsequently, after appropriate dilution with mobile phase, they are analyzed by HPLC-UV (Hitachi 11 Chromaster; Hitachi Ltd., Tokyo, Japan or Spectra System HPLC, ThermoQuest Inc., San Jose, USA). A BDS Hypersil C18, 5 µm, 150 x 4.6 mm (Thermo Scientific) analytical column combined with a precolumn (BDS Hypersil C-18, 3µm, 10 x 4mm) is used. The mobile phase consists of 20 mM NaH2PO4 buffer adjusted to pH=3.0 and acetonitrile (60:40 % v/v). The detection wavelength is set at 273 nm, the flow rate at 1.2 mL/min and the injection volume at 20 µL. Using this method, the retention time is approximately 7.3 minutes. The limit of detection (LOD) and quantification (LOQ) are 0.03 and 0.1 µg/mL, respectively.

Once confidence in the estimation of solubility-related parameters is established, further model based analysis of the in vitro dissolution data obtained from both the one and two-stage tests is performed within the serial dilution module of the SIVA Toolkit^{®} (SIVA 3.0). The dissolution rate of spherical particles under sink and non-sink conditions within SIVA is described by an extension of the diffusion layer model (DLM) developed by Wang and Flanagan (1999, General solution for diffusion-controlled dissolution of spherical particles. 1. Theory, J.Pharm.Sci., 88(7), 731-8).

By fitting the DLM model to the observed dissolution data, accurate estimates for each dissolution and two-stage test are obtained. In the case of two-stage testing, the gastric and intestinal profiles are treated separately. Under fasted state intestinal conditions, Zn(II)-NSAID-complex is freely soluble and therefore in vitro dissolution is not expected to be solubility limited. In that case, disintegration of the solid dosage form in the intestinal dissolution medium might be the rate-limiting step for the in vitro dissolution rate, especially in single dissolution experiments where the dosage form is directly exposed to the intestinal medium without any pre-treatment with gastric medium to account for disintegration in the stomach. In order to distinguish and model the relative impact of disintegration on the overall dissolution, the first-order disintegration option is activated in SIVA and used to obtain estimates of the first-order disintegration rate constant for these experiments. In the case of intestinal dissolution profiles generated after two-stage testing, the first order disintegration option is deactivated since disintegration in the stomach had been already accounted for by the dissolution in the gastric medium. For dissolution experiments of the pure drug, the disintegration time is assumed to be negligible. Estimation of the relevant parameters is performed using the Nelder-Mead algorithm and equal weighting is applied.

### Example 3: Dissolution testing of nanoemulsions

The following Zn(II)-NSAID complexes are investigated:
- Zn(II)-Mefenamin
- Zn(II)-Ibuprofen
- Zn(II)-Flurbiprofen
- Zn(II)-Diclofenac
- Zn(II)-NO-Ibuprofen
- Zn(II)-NO-Flurbiprofen

Nanoemulsions are prepared by means of an opportunely modified solvent evaporation method (QESD) (Kawashima et al., 1989), to achieve particles with size, charge and stability features suitable for ocular applications.

The nanoemulsions are obtained in the presence or absence of NSAID, at different drug/polymer weight ratio and using different rates of agitation. Drug and polymer are co-dissolved at room temperature in ethanol (2 ml). The solution is slowly injected (0.5 ml/min), with a syringe connected to a thin teflon tube, in 50 ml water containing Tween 80 (0.02% w/v) and benzalkonium chloride (0.1% w/v), and kept in an iced-water bath. During injection, the mixture is highly mixed by an Ultra-Turrax T25 at agitation speed 9.500rpm, 20.500rpm and 24.000rpm.

The solution immediately turns into a pseudoemulsion of the drug and polymer ethanol solution in the external aqueous phase. The counter-diffusion of ethanol and water out of and into the emulsion microdroplets, respectively, and the gradual evaporation of the organic solvent determine the precipitation in situ of the polymer and drug, with the formation of matrixtype nanoparticles. Ethanol residues evaporate off during a further slow magnetic stirring (24 h) of the nanoemulsion at room temperature.

In order to verify the effect of pH of the preparation medium on drug incorporation and successive release, an extra formulation is also prepared by using two different phosphate buffers (at pH 7.4 or 5.5), as the dispersing medium. Similarly, to investigate the influence of the different water permeability of the two polymers, a mixed system (batch 9) is obtained where the polymeric matrix consists of equivalent amounts of RS and RL, using a pH 7.4 phosphate buffer as the dispersing medium.

Two-ml aliquots of the freshly prepared suspensions are centrifuged at 11,000 rpm and at 101 C for 15 min (IEC/Centra MP 4R centrifuge equipped with an 851-type rotor), and the amount of unincorporated drug is measured by UV analysis in the supernatant. Some supernatant samples are submitted to a second centrifugation cycle, but no solid material could be further collected.

Drug crystalline state in the polymer systems is evaluated in the freeze-dried samples by powder X-ray diffractometry and IR analysis. X-ray spectra are recorded with a Philips PW 1050/25 diffractometer for powders. A voltage of 40 kV and a current of 30mA for the generator are used, with Cu as the tube anode material. The solids are exposed to a Cu-Kalpha radiation, over a range of different angles, at a predefined angular speed, using divergence and receiving slits.

IR spectra of freeze-dried nanoparticles are obtained with a Perkin-Elmer 1600 spectrophotometer, using the KBr disk technique (about 10 mg sample for 100 mg dry KBr). The mean particle size of the formulations is determined by photo-correlation spectroscopy with a Zetamaster (Malvern Instruments Ltd., Worcs, UK) equipped with the Malvern PCS software. Every sample is appropriately diluted with 0.45 µm-filtered water and the reading is carried out at a 90 degree angle in respect to the incident beam.

The release of NSAID from nanoemulsions is evaluated over 3 h by a dialysis system consisting of a Spectrapor membrane (cut-off: 3500 Da), loaded with 5 ml of nanoemulsion and soaked in a 0.14 M phosphate buffer solution (pH 7.4), at room temperature and under slow magnetic stirring. At regular intervals aliquots of 1 ml of the dissolving medium are withdrawn, and immediately restored with the same volume of fresh buffer. The amount of released drug is assessed by UV analysis at 247nm (Shimadzu UV-1601). To check the eventual limiting effects of dialysis membrane on drug dissolution, separate experiments are performed in duplicates with a solution of pure NSAID in the same phosphate buffer, with two of the drug concentrations used in the nanoemulsions. Similarly, an aqueous suspension of powdered drug (250-µm sieve) is loaded into the dialysis bag and the test is carried out (in duplicates).

Aliquots of different batches are freeze-dried to verify the physical stability and the following redispersibility. Aliquots of 10 ml are taken, frozen in liquid nitrogen and lyophilised in an Edward instrument (Freeze-Dryer Modulyo) for 24 h at 30 °C at a pressure of 0.05 mmHg.

Every 2-month intervals the freeze-dried samples, stored at room temperature, were rehydrated with the original volume of distilled water to restore the drug and polymer concentrations. Size and x -potential changes are assessed as described above. Reconstituted samples are also centrifuged as described above, and the drug leakage is determined in the supernatant by UV analysis.

The physical stability of the nanoemulsion is evaluated after storage for 18 months under different temperature conditions. Exact volumes of each nano-suspension are stored in closed glass bottles and placed at room temperature or at 4-6 °C (refrigerator) out from direct light. Aliquots of 500 µl are withdrawn every 2 months to determine particle size, and potential drug leakage.

### Example 4: In vivo testing of ocular nanoemulsion

The following Zn(II)-NSAID complexes are investigated:
- Zn(II)-Flurbiprofen (0,03%/ 0,154mg)
- Zn(II)-Diclofenac (1,028mg)
- Zn(II)-NO-Flurbiprofen (0,03%/ 0,154mg)

Pharmacological tests are performed on groups of three female New Zealand albine rabbits, 1.8-2.2 kg b.w., without any inflammation symptom or gross abnormality. The experimental procedures are in agreement with the ARVO guide-lines (Association for Research in Vision and Ophtalmology).

The formulation, diluted with saline to a final NSAID concentration of 0.03% for Flurbiprofen and (w/v), the ARS100C batch (empty RS nanosuspension) or the commercial reference product (Ocufens Allergan; Diclobac), containing the NSAID in above dosage (w/v), are instilled (50 ml) in the conjunctive sac of rabbit left-eye 180, 120, 90, and 30 min before, and then 5, 15, 75, and 90 min after the first paracentesis.

Right eye of each animal is used as a control. Fifty microlitres of a 1% atropine solution is applied to both the treated and control eyes 90 min before the first paracentesis.

To perform the paracentesis, animals are lightly anaesthetised with an i.v. injection of ketamine hydro-chloride (50 mg/kg b.w.). Aqueous humour samples for each animal are collected with a 26-gauge needle attached to a tuberculin syringe. The needle is introduced into the anterior chamber through the cornea, taking care not to damage the iris, the lens and the anterior uvea; eye conditions are carefully examined using a slit lamp every hour after the paracentesis. Fifty microlitres of aqueous humour are removed and analysed by HPLC for drug concentration. The pupil diameter of the treated and control eye is measured with a Castroviejo calipe 180 and 5 min before the first paracentesis and then 5 min before the second one. At the end of the treatment schedule, the animals are killed by i.v. injection of 0.3 ml kg-1 Tanax.

The potential ocular irritancy and/or damaging effects of formulations are evaluated according to a modified Draize test using a slit-lamp.

Observation of the ocular tissue condition is performed after 10 min, 6 and 24 h after the end of the experiments.

The congestion, swelling and discharge of the conjunctiva are graded on a scale from 0 to 3, 0 to 4 and 0 to 3, respectively.

In this, hyperemia and corneal opacity are graded on a scale from 0 to 4. Formulations (50 ml) are topically administered in the right eye every 30 min for 6 h (12 treatments). At the end of the treatment, three observations at 10 min, 6 h and 24 h are carried out to evaluate the ocular tissues. Drug concentration in the aqueous humour of the treated eyes is measured by HPLC, against a calibration curve obtained from known amount of NSAID (external standard). A Hypersil ODS C18 column is used. The eluent phase (1 ml/min) consists of a 65:35 (v/v) mixture of 0.1 m sodium acetate (pH 6.31) and acetonitrile. Samples are read at 278 nm.

Aqueous humour samples are added with 6% perchloric acid to denature proteins and filtered through a 0.45 mm Spartan Teflon membrane before injection in the instrument. The recovery efficiency from blank rabbit aqueous humour to which 20 and 5 µgml-1 of a FLU standard have been added is 97.2% and 93.8%, respectively.

## Claims

1. A pharmaceutical composition comprising
i) a Zn(II)-complex of a non-steroidal anti-inflammatory drug (NSAID) selected from the group consisting of any one of aspirin (acetylsalicylic acid), flurbiprofen, diclofenac, mefenamic acid, dexibuprofen, and ibuprofen (Zn(II)-NSAID-complex), optionally further comprising nitric oxide (Zn(II)-NO-NSAID-complex),
ii) a hydrophilic surfactant, specifically selected from the group consisting of PEG 300, PEG 400, PEG15, polyoxyethylene (20) cetyl ether, ethanol, propylene glycol, glycerol, and diethylene glycol monoethyl ether, and
iii) one or more mucoadhesive agents.

2. The pharmaceutical composition of claim 1, wherein the mucoadhesive agent is selected from the groups consisting of:
I. natural polymers, specifically hyaluronate, pectin, alginate, xanthin gum, and guar gum,
II. cyclodextrins, specifically α cyclodextrin, β cyclodextrin, γ cyclodextrin, and methylated β-cyclodextrins,
III. hydrogen bond polymers, specifically acrylate, more specifically hydroxylated methacrylate poly(methacrylic acid), butylmethacrylate, poly[acrylic acid-co-(2-ethylhexyl)acrylate-co-methylacrylate], high molecular weight crosslinked polyacrylic acid polymers, and polycarbophil (PC),
IV. cationic compounds, specifically poly-I-arginine and l-lysine,
V. non-ionic polymers, specifically hydroxyethyl starch, hydroxypropyl cellulose (HPC), poly(ethylene oxide), polyvinyl alcohol (PVA), polyvinylpyrrolidone (PVP), scleroglucan, polyoxyethylene poly (methacrylic acid co-methyl methacrylate), and
VI. positively charged polymers, specifically chitosan, trimethylchitosan, aminodextran, dimethylaminoethyl (DMAE), diethylaminoethyl (DEAE), dextran.

3. The pharmaceutical composition of claim 1 or 2, further comprising NSAIDs and related compounds, specifically selected from the group consisting of ibuprofen, ibuprofen lysine, ibuprofen arginate, ibuprofen sodium salt, ibuprofen calcium salt, mefenamic acid, sodium mephenamate, mefenamin-lysinate, mefenamin-arginate, dexibuprofen, dexibuprofen lysine, diclofenac sodium salt, flurbiprofen, flurbiprofen sodium salt, aspirin, aspirin calcium, aspirin sodium salt, and aspirin DL-lysine.

4. The pharmaceutical composition of any one of claims 1 to 3, further comprising one or more of calcium ascorbate, caffeine, caffeine citrate, chlorpheniramine maleate, D-pseudoephedrine, or pseudoephedrine sulfate.

5. An emulsion comprising
a. a Zn(II)-NSAID-complex or Zn(II)-NO-NSAID-complex of any one of claims 1 to 4,
b. a co-surfactant,
c. an oil, and
d. an aqueous solution.

6. The emulsion of claim 5, wherein the emulsion is a water in oil, an oil in water, or a bicontinuous emulsion.

7. The emulsion of claim 5 or 6, comprising droplets, with a droplet size in the range of 10 to 1000 nm, specifically of 10 to 500 nm, specifically of 10 to 200 nm, specifically of 10 to 150 nm.

8. The emulsion of any one of claims 5 to 7, wherein a co-surfactant is selected from the group of nonionic tensides, specifically polyoxylglycerides, esters of fatty acids and alcohols, or sucrose ester-based natural surfactants.

9. The emulsion of any one of claims 5 to 8, wherein the oil is selected from the group of vegetable oils, hydrogenated vegetable oils, or partial glycerides, more specifically corn oil, olive oil, peanut oil, rapeseed oil, sesame oil, soybean oil, coconut, palm seed oil, cottonseed oil.

10. The emulsion of any one claims 5 to 9, further comprising dexpanthenol.

11. The pharmaceutical composition of any one of claims 1 to 4, or the emulsion of any one of claims 5 to 10, formulated for local administration, preferably for intraoral, oral, dermal, ocular administration, for systemic administration, for rectal administration, for vaginal administration, application to the upper and lower respiratory tract, nasal administration, or pulmonary administration.

12. The pharmaceutical composition of any one of claims 1 to 4, or the emulsion of any one of claims 5 to 10, for administration as a spray, a powder, a gel, an ointment, a cream, a foam, or a liquid solution, a lotion, a gargle solution, an aerosolized powder, an aerosolized liquid formulation, a granule, a capsule, a drop, a tablet, a troche, a syrup, a lozenge, a solid or semi-solid candy, an eye drop, a gum, a suppository, a transdermal patch or a preparation for infusion or injection.

13. The pharmaceutical composition of any one of claims 1 to 4, or an emulsion of any one of claims 5 to 10 for use in treating pain, inflammation, problems associated to wound healing, and/or infection with gram negative, gram-positive bacteria or yeast.

14. The pharmaceutical composition of any one of claims 1 to 4, or an emulsion of any one of claims 5 to 10 for use as analgesic, anti-pyretic or anti-inflammatory agent.

15. The pharmaceutical composition of any one of claims 1 to 4, or an emulsion of any one of claims 5 to 10 for use in inhibiting growth of gram-positive, gram-negative bacteria or yeasts in a subject.
